# EUROPEAN PATENT APPLICATION

(11) **EP 4 265 215 A1**
(43) Date of publication of application: **25.10.2023**
(21) Application number: 21904936.8
(22) Date of filing: 10.05.2021
(51) Int. Cl.: A61B 34/30

(54) **SURGICAL ROBOT, AND CONTROL METHOD AND CONTROL APPARATUS THEREFOR**

(30) Priority: 15.12.2020 CN 202011472823
(71) Applicant: Shenzhen Edge Medical Co., Ltd., Shenzhen, Guangdong 518000 (CN)
(72) Inventor: GAO, Yuanqian, Shenzhen, Guangdong 518000 (CN); WANG, Jianchen, Shenzhen, Guangdong 518000 (CN)
(74) Representative: Biallo, Dario
(86) International application number: PCT/CN2021/092570
(87) International publication number: WO 2022/126997

(57) **Abstract**

A surgical robot, and a control method and control apparatus therefor. The control method comprises: acquiring a position limits of a controlled-end device; determining whether the current position of the controlled-end device reaches the position limits; when the current position of the controlled-end device reaches the position limits, controlling the controlled-end device to maintain the current position, and controlling the posture of the controlled-end device to change according to a posture instruction inputted by a motion input device; otherwise, controlling the position and posture of the controlled-end device to change according to a position instruction and the posture instruction that are inputted by the motion input device. According the control method, on one hand, the safety and reliability of surgery can be ensured by means of allowing the controlled-end device to move within the position limits and maintain the position thereof when the controlled-end device reaches the position limits; and on the other hand, when the controlled-end device reaches the position limits, the posture of the controlled-end device changes according to the posture instruction, and there is no need to re-align the postures of the motion input device and the controlled-end device, thereby ensuring surgical continuity.

## Description

The present disclosure claims the benefit of Chinese Patent Application NO. 202011472823.6, entitled "surgical robot, and control method and control apparatus thereof", which was filed with China National Intellectual Property Administration on December 15, 2020, and the entire contents of which are incorporated herein by reference.

### FIELD

The present disclosure relates to medical instruments, more particularly to a surgical robot, and control method and control apparatus thereof.

### BACKGROUND

Minimally invasive surgery refers to a surgical method for performing surgery in a human body cavity using modern medical instruments such as laparoscopes, thoracoscopes, and related devices. Compared with traditional surgery modes, minimally invasive surgery has advantages of resulting in small trauma, light pain, fast recovery, and etc.

With advances in science and technology, minimally invasive surgical robotics are increasingly mature and widely used. A surgical robot includes a master control console and a slave operating device, the slave operating device includes a plurality of operating arms, and the plurality of operating arms include a camera arm with an image end effector and a surgical arm with an operation end effector. The master control console includes a display and a handle. By operating the handle, a doctor controls the movement of the camera arm or the surgical arm in the field of view provided by the camera arm and displayed on the display.

Due to the physical position-limit and/or the software position-limit to the slave operating device, when the end effector of the operating arm triggers the position-limit, generally, the end effector maintains the pose to ensure the safety and the reliability. But the handle which controls the movement of the end effector may not trigger a corresponding position-limit, that is, the handle may probably move unrestrictedly. In this case, an operational process of aligning the orientations may be necessary when restarting to control the end effector after having released the position-limit, which causes discontinuities in the surgical operation.

### SUMMARY

In view of the above, the present disclosure provides a surgical robot, and control method and control apparatus thereof, which ensures the safety, reliability, and continuity in a surgical operation.

A first aspect of the present disclosure provides a control method of a surgical robot, the surgical robot includes an actuating arm and a motion input device for manipulating changes in pose of a distal end of a first portion of the actuating arm, the distal end of the first portion includes an end effector, the end effector includes a controlled end effector currently configured to be manipulated by the motion input device, the control method includes steps of: obtaining a position-limit of the controlled end effector; determining whether a current position of the controlled end effector reaches the position-limit; and controlling the controlled end effector to maintain the current position, and controlling an orientation of the controlled end effector to change by following an orientation instruction inputted by the motion input device, in case where the current position of the controlled end effector reaches the position-limit; otherwise, controlling the position and the orientation of the controlled end effector to change by following an position instruction and an orientation instruction inputted by the motion input device.

In one embodiment, the position-limit is a position-limit in a task space, the step of determining whether the current position of the controlled end effector reaches the position-limit, includes: obtaining the current position of the controlled end effector in the task space in real time; and determining whether the controlled end effector reaches the position-limit by determining whether the current position reaches the position-limit.

In one embodiment, the position-limit is a maximum range of motion of the controlled end effector allowed by coordinative movements of respective joint assemblies in the first portion.

In one embodiment, the position-limit is a range of motion within the maximum range of motion that is determined based on the maximum range of motion.

In one embodiment, the changes in pose of the controlled end effector are determined according to movements of respective joint assemblies in the first portion, the position-limit is a position-limit of each assembly in the first portion in a joint space, the step of determining whether the current position of the controlled end effector reaches the position-limit includes: obtaining joint variables of each joint assembly in the first portion in real time; and determining whether the controlled end effector reaches the position-limit by determining whether the joint variables corresponding to each joint assembly reach the position-limit.

In one embodiment, the position-limit is a position limit of each joint assembly in the first portion that affects the change in position of the controlled end effector.

In one embodiment, the controlled end effector is an image end effector.

In one embodiment, the controlled end effector is an operation end effector.

In one embodiment, the position-limit is determined based on a visible area of the image end effector in a reference frame.

In one embodiment, the position-limit is the visible area of the image end effector in the reference frame.

In one embodiment, the position-limit is an area within the visible area.

In one embodiment, the visible area is determined based on camera parameters of the image end effector, and the camera parameters comprise a field angle and a depth of field.

In one embodiment, the step of determining whether the controlled end effector reaches the position-limit includes: obtaining an operation image of the visible area captured by the image end effector; and determining whether the controlled end effector reaches the position-limit by recognizing whether the controlled end effector is in the operation image.

In one embodiment, the step of controlling the position and the orientation of the controlled end effector to change by following position instructions and orientation instructions inputted by the motion input device includes: obtaining a safe area of motion within the visible area, defining an area within the safe area of motion as a first area, and defining an area outside of the safe area of motion and within the visible area as a second area; and changing movement speed of the controlled end effector according to the changes in position and in movement direction of the controlled end effector in the first area and the second area.

In one embodiment, the step of changing the movement speed of the controlled end effector according to the changes in position and in movement direction of the controlled end effector in the first area and the second area includes: decreasing the movement speed of the controlled end effector in a corresponding direction in case where the controlled end effector moves from a boundary of the first area to an outer boundary of the second area; and increasing the movement speed of the controlled end effector in a corresponding direction in case where the controlled end effector moves from the outer boundary of the second area to the boundary of the first area.

In one embodiment, the movement speed of the controlled end effector in the corresponding direction is positive correlation with a distance between the controlled end effector and the outer boundary of the second area.

In one embodiment, the motion input device is a mechanical motion input device, and the step of controlling the position and the orientation of the controlled end effector to change by following position instructions and orientation instructions inputted by the motion input device includes:

obtaining a safe range of motion within the visible area, defining an area within the safe range of motion as a first area, and defining an area outside of the safe range of motion and within the visible area as a second area; and increasing a resistance force against the controlled end effector moving in a corresponding direction in case where the controlled end effector moves from a boundary of the first area to an outer boundary of the second area; and decreasing the resistance force to the controlled end effector moving in the corresponding direction in case where the controlled end effector moves from the outer boundary of the second area to the boundary of the first area.

In one embodiment, the resistance force against the motion input device moving in the corresponding direction is in negative correlation with the distance between the controlled end effector and the outer boundary of the second area.

In one embodiment, the control method includes: obtaining description information of configuration of the actuating arm; and generating a configuration interface for configuring the first portion based on the description information, the configuration interface comprising a control which is associated with structure of each part of the actuating arm.

In one embodiment, the configuration interface includes a model image generated based on the description information and associated with the actuating arm, and the model image comprises a control corresponding to each part of the actuating arm or a control corresponding to each joint assembly of each part of the actuating arm.

Another aspect of the present disclosure provides a computer-readable storage medium, the computer-readable storage medium stores a computer program, which is configured for being loaded and executed by a processor to implement the steps of the control method according to any one of the above embodiments.

A further aspect of the present disclosure provides control apparatus of surgical robot, which includes: a memory, configured for storing a computer program; and a processer, configured for loading and executing the computer program; in which the computer program is configured for being loaded and executed by the processor to implement the steps of the control method according to any one of the above embodiments.

A further aspect of the present disclosure provides a surgical robot, which includes: an actuating arm; a motion input device for manipulating changes in pose of a distal end of a first portion of the actuating arm; and a controller coupled to the actuating arm and the motion input device, and configured for implementing the steps in the control method according to any one of the above embodiments.

In one embodiment, the actuating arm includes a mechanical arm and an operating arm, the operating arm is mounted to a distal end of the mechanical arm, the end effector is mounted to a distal end of the operating arm; and the first portion is the operating arm, or, the first portion is the mechanical arm and the operating arm.

In one embodiment, the actuating arm includes a mechanical arm, an adjusting arm, a manipulator and an operating arm, a proximal end of the adjusting arm is mounted to a distal end of the mechanical arm, a proximal end of the manipulator is mounted to a distal end of the adjusting arm, a proximal end of the operating arm is mounted to a distal end of the manipulator, and the end effector is mounted to a distal end of the operating arm; the first portion is the operating arm, or, the first portion is the manipulator and the operating arm, or, the first portion is the mechanical arm, the adjusting arm, the manipulator and the operating arm.

The surgical robot, and control method and control apparatus thereof of the present disclosure have the following beneficial effects:

On one hand, by allowing the controlled end effector to move within the position-limit when the controlled end effector does not reach the position-limit, and by controlling the controlled end effector to maintain the position when the controlled end effector reaches the position-limit, the safety and reliability of the surgical operation may be ensured;

On the other hand, by controlling the orientation of the controlled end effector to change by following the orientation instructions inputted by the motion input device, the motion input device always maintains the same orientation with the controlled end effector, so that it is no need to re-align the two in orientation, and the continuity in the surgical operation may be ensured.

### BRIEF DESCRIPTION OF THE DRAWINGS

FIG. 1 is a schematic structure diagram of a surgical robot according to an embodiment of the present disclosure;
FIG. 2 is a partial schematic diagram of the surgical robot shown in FIG. 1 according to an embodiment of the present disclosure;
FIG. 3 is a schematic structure diagram of an operating arm and a power device of the surgical robot;
FIG. 4 is a schematic structure diagram of a surgical robot according to another embodiment of the present disclosure;
FIG. 5 is a configuration interface for configuring a first portion of the surgical robot shown in FIG. 1 according to an embodiment of the present disclosure;
FIG. 6 is a configuration interface for configuring a first portion of the surgical robot shown in FIG. 1 according to another embodiment of the present disclosure;
FIG. 7 is a flow chart of a control method of a surgical robot according to an embodiment of the present disclosure;
FIG. 8 is a principal diagram of the control method shown in FIG. 7 according to an embodiment of the present disclosure;
FIG. 9 to FIG. 12 are flow charts of the control method of the surgical robot according to different embodiments of the present disclosure;
FIG. 13 is a partial schematic diagram of the surgical robot under an operation state;
FIG. 14 is a schematic diagram of a controlled end effector moving in different areas;
FIG. 15 is a flow diagram of a control method of a surgical robot according to an embodiment of the present disclosure;
FIG. 16 is a schematic structure diagram of a control apparatus of a surgical robot according to an embodiment of the present disclosure.

### DETAILED DESCRIPTION

In order to facilitate the understanding of the present disclosure, the present disclosure will be more fully described below with reference to the relevant accompanying drawings. Preferred embodiments of the present disclosure are given in the accompanying drawings. However, the present disclosure may be implemented in many different manners and is not limited to the embodiments described herein. Rather, these embodiments are provided for the purpose of providing a more thorough and comprehensive understanding of the disclosed contents of the present disclosure.

It should be noted that when an element is referred to as being "disposed on" another element, it may be directly on the other element or intervening elements may also be present. When an element is considered to be "connected" to another element, it may be directly connected to another element or intervening elements may be present at the same time. When an element is considered to be "coupled" to another element, it may be directly coupled to another element or intervening elements may be present at the same time. As used herein, the terms "vertical", "horizontal", "left", "right" and the like are intended for purposes of illustration only and are not intended to be limiting. As used herein, the terms "distal end" and "proximal end" are common terms in the art of interventional medical devices, where "distal end" refers to the end far away from the operator during the surgical procedure, and the "proximal end" refers to the end close to the operator during the surgical procedure. The term "first/second" and the like as used in this disclosure indicates a component and a group of two or more components having common characteristics.

Unless defined otherwise, all technical and scientific terms used herein have the same meaning as commonly understood by one of ordinary skill in the art. The terminology used herein is for the purpose of describing particular embodiments only and is not intended to be limiting of the disclosure. As used herein, the term "and/or" includes any and all combinations of one or more of the relevant listed items. As used in this disclosure, the term "each" includes one or more than two.

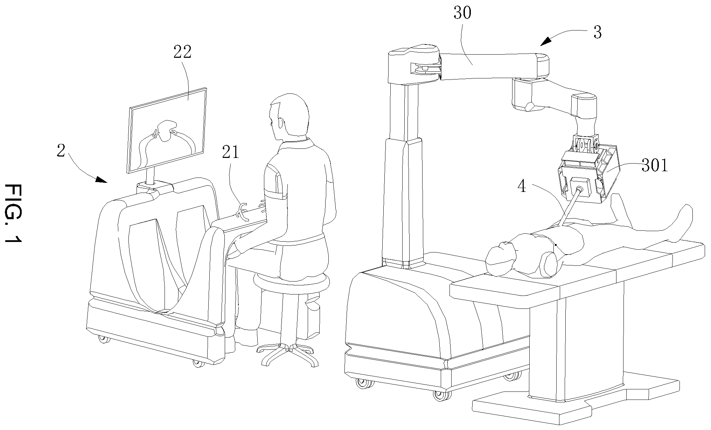

Refer to FIG. 1 to FIG. 2, which are the schematic structure diagram and the partial diagram of a surgical robot according to an embodiment of this disclosure.

The surgical robot includes a master control console 2 and a slave operating device 3 controlled by the master control console 2. The master control console 2 includes a motion input device 21 and a display 22, and a doctor sends control commands to the slave operation device 3 by operating the motion input device 21, so that the slave operating device 3 performs corresponding operations according to the control commands sent by the doctor by controlling the motion input device 21, and the doctor observes the surgical area through the display 22. In this case, the slave operating device 3 includes an actuating arm, and the actuating arm includes a mechanical arm 30 and at least one operating arm 31 detachably mounted to the distal end of the mechanical arm 30. The mechanical arm 30 includes a base and a connecting assembly, which are sequentially connected, and the connecting assembly includes a plurality of joint assemblies. The operating arm 31 includes a link 32, a connecting assembly 33, and an end effector 34, which are sequentially connected, where, the connecting assembly 33 includes a plurality of joint assemblies, and the orientation of the end effector 34 is adjusted by adjusting the joint assemblies of the operating arm 31. The end effector 34 includes an image end effector 34A and an operation end effector 34B. The image end effector 34A is configured for capturing the images within the field of view, and the display 22 is configured for displaying the images. The operation end effector 34B is configured for performing surgical operation such as cutting, suturing. In the disclosure, the operating arm with the image end effector 34A is named as a camera arm 31A, and the operating arm with the operation end effector 34B is named as a surgical arm 31B.

The surgical robot shown in FIG. 1 is a single-port surgical robot, in which each operating arm 31 may insert into the patient's body through a same puncture device 4 mounted to the distal end of the mechanical arm 30. In the single-port surgical robot, the doctor generally only controls the operating arm 31 to complete basic surgical operations. In this case, the operating arm 31 of the single-port surgical robot should have both position-related DoFs (that is, degrees of freedom for determining position) and orientation-related DoFs (that is, degrees of freedom for determining orientation), so as to realize the change in pose within a certain range. For example, the operating arm 31 may have a DoF x of horizontal movement, a DoF y of vertical movement, a DoF α of roll, a DoF β of pitch, and a DoF γ of yaw. The operating arm 31 may also realize a DoF z of forward and backward movement (that is, degree of freedom for feeding) under the actuation of the distal joint assemblies (i.e., the power mechanism 301) of the mechanical arm 30. In addition, in some embodiments, at least one redundant DoF may also be set for the operating arm 31 to realize the possibility of more functions. For example, under the condition that the six DoFs have been realized, additional one, two, or more DoFs may be set. For example, the power mechanism 301 may include a guide rail and a power device slidably arranged on the guide rail. The operating arm 31 is detachably mounted on the power device. On one hand, the slide of the power device on the guide rail provides the DoF z of forward and backward movement of the operating arm 31, and on the other hand, the power device provides the power for the joint assemblies of the operating arm 31 to realize the other five DoFs (that is, [x, y, α, β, γ]).

The surgical robot may further include a controller. The controller may be integrated in the master control console 2 or integrated in the slave operating device 3. Or, the controller may be independent from the master control console 2 and the slave operating device 3. For example, the controller may be deployed locally, or deployed in the cloud. The controller may include at least one processor.

The surgical robot may further include an input device. The input device may be integrated in the master control console 2 or integrated in the slave operating device 3. Or, the input device may be independent from the master control console 2 and the slave operating device 3. The input device may be for example a mouse, a keyboard, a voice input device, or a touch screen. In one embodiment, a touch screen may be used as the input device. For example, the touch screen may be arranged at an armrest of the master control console 2.

The operating arm 31 may further include sensors for sensing the joint variables of the joint assemblies. The sensors may include angle sensors and displacement sensors, the angle sensors are configured to sense the rotational motion of the joint assemblies, and the displacement sensors are configured to sense the linear motion of the joint assemblies. Specifically, appropriate sensors may be configured according to the types of the joint assemblies.

The controller may be coupled to the sensors, and to the input device and the display 22.

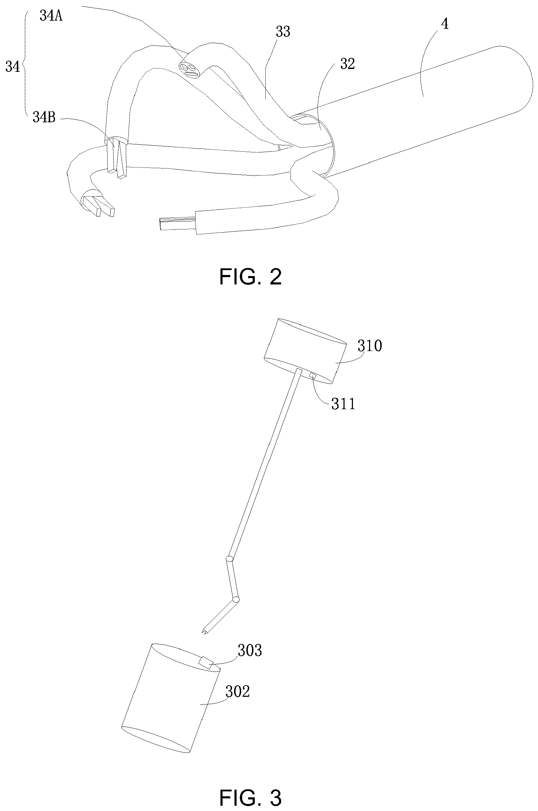

For example, referring to FIG. 3, a storage unit 311 may be arranged at an abutting surface of the actuating box 310 of the operating arm 31, which abutting surface of the actuating box 310 abuts the power device 302 of the power mechanism 301, and a reading unit 303 matching the storage unit 311 is arranged at an abutting surface of the power device 302, which abutting surface of the power device 302 abuts the actuating box 310. The reading unit 303 may be coupled to the controller. In case where the operating arm 31 is mounted to the power device 302, the reading unit 303 may communicate with the storage unit 311 and read relevant information from the storage unit 311. The storage unit 311 may be for example a memory, or an electronic tag. The storage unit may store for example the type of the operating arm, the part(s) of the operating arm which may be configured as the target part(s), and a kinematic model of the operating arm, and etc. For example, the storage unit 311 of the camera arm 31A may further store additional camera parameters.

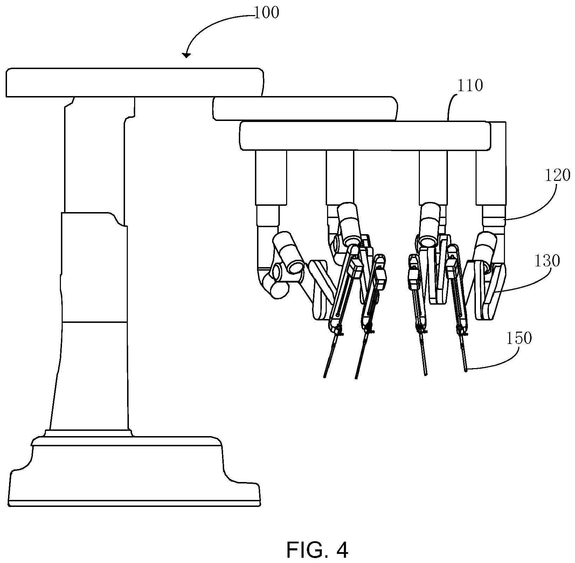

Refer to FIG. 4, which is a schematic structure diagram of a surgical robot according to an embodiment of the present disclosure. More particularly, FIG. 4 illustrates a schematic structure diagram of a multi-port surgical robot according to an embodiment of the present disclosure. Difference between the multi-port surgical robot shown in FIG. 4 and the single-port surgical robot shown in FIG. 1 mainly lies in the difference between the slave operating devices of the two. The actuating arm of the multi-port surgical robot shown in FIG. 4 includes a mechanical arm 110, an adjusting arm 120, a manipulator 130, and an operating arm 150, which are sequentially connected. The numbers of the adjusting arms 120 and the manipulators 130 are the same and more than two, for example, may be four. The distal end of the mechanical arm 110 includes an orienting platform, the proximal ends of the adjusting arms 120 are connected to the orienting platform, and the proximal ends of the manipulators 130 are connected to the distal ends of the adjusting arms 120. The manipulators 130 may be configured to detachably connect to the operating arm 150, and each manipulator 130 includes a plurality of joint assemblies. In the multi-port surgical robot, different operating arms 150 may insert into the patient's body with different puncture devices. Compared with the operating arm 31 of the single-port surgical robot, generally, the operating arm 150 of the multi-port surgical robot includes fewer DoFs. Generally, the operating arm 150 includes the orientation-related DoFs (i.e., degrees of freedom for determining orientation) only, and of course the changes in the orientation generally may affect the position, but the effect can be ignored as it is tiny in some situation. The change in the position of the operating arm 150 generally can be realized with the assistance of the manipulator 130. Because the changes in the pose are realized by the associated actions of the manipulator 130 and the operating arm 150, the two may be named as a manipulator assembly, which is equivalent to the operating arm 31 of the single-port surgical robot.

Depending on the configuration, the motion input device 21 may input pose instructions including position instructions and orientation instructions to control the changes in the pose of the distal end of a first portion of the actuating arm. The distal end of the first portion may usually refer to an end effector. In addition, the distal end of the first portion may also refer to a joint assembly which connected to the end effector, and the changes in the pose of the end effector is in consistence with the changes in the pose of the joint assembly.

In the surgical robot shown in FIG. 1, the actuating arm may include the mechanical arm and the operating arm, the proximal end of the operating arm is mounted to the distal end of the mechanical arm, and the end effector is mounted to the distal end of the operating arm. Depending on the configuration, the first portion may be configured as the operating arm, or, the first portion may be configured as the mechanical arm and the operating arm.

Correspondingly, in the surgical robot shown in FIG. 4, the actuating arm may include the mechanical arm, the adjusting arm, the manipulator, and the operating arm, the proximal end of the adjusting arm is mounted to the distal end of the mechanical arm, the proximal end of the manipulator is mounted to the distal end of the adjusting arm, the proximal end of the operating arm is mounted to the distal end of the manipulator, and the end effector is mounted to the distal end of the operating arm. Depending on the configuration, the first portion may be configured as the operating arm, or, the first portion may be configured as the manipulator and the operating arm, or, the first portion may be configured as the mechanical arm, the adjusting arm, the manipulator and the operating arm.

It is understandable that no matter it is the single-port surgical robot shown in FIG. 1 or the multi-port surgical robot shown in FIG. 4, the mechanical arm is generally configured to adjust the pose of the end effector in a large range, and the operating arm is generally configured to finely adjust the pose of the end effector. For example, the mechanism arm may be configured to set the position before the surgical operation, and the operation may be mainly realized by controlling the operating arm during the operation process. Certainly, in some embodiments, some specific function may be realized by combining the mechanism arm, the operating arm, and the corresponding arm structure to move coordinately.

In one embodiment, the structure expected to be associated with the first portion of the actuating arm can be defined in the system files of the surgical robot, and the structure associated with the first portion may be read from the system files when the system of the surgical robot is initialized and it can be applied in the following embodiments.

In one embodiment, a configuration interface for configuring the first portion may also be generated in real time according to the description information of the configuration of the actuating arm. The description information includes the link parameters of all of the joint assemblies of each portion of the actuating arm, and etc. For example, the configuration interface may include a control(s) for the doctor to configure, the control(s) may be associated with the structure of each portion of the actuating arm and may be available for selection. For example, the control may be a text control, an optional control such as a drop-down list control, a button control, and others.

Preferably, in order to facilitate the doctor to configure the first portion through the configuration interface more intuitively, a model image may also be generated according to the description information of the configuration of the actuating arm, and the model image is associated with the description information of the configuration of the actuating arm and includes controls available for selection. The model image may be a concise projection model image, or a complicated structured computer model. The model image may change with the changes of the states of the actuating arm. Certainly, the model image may also not to follow the changes of the states of the actuating arm, and for example only present the initial states (in case where, for example, the joint variables is zero) of the configuration of the actuating arm at a certain time. The control of the model image may be an icon control, specifically, it may be a light spot, an aperture, and the like.

For example, for the surgical robot shown in FIG. 1, the mechanism arm and the operating arm of the actuating arm may respectively correspond to a control for selecting them together to be the first portion. For the surgical robot shown in FIG. 4, the mechanical arm, the adjusting arm, the manipulator and the operating arm of the actuating arm may respectively correspond to a control for selecting them together to be the first portion.

For example, for the surgical robot shown in FIG. 1 to FIG. 4, each joint assembly of the actuating arm may respectively correspond to a control for selecting a partial or all of them to be the first portion. In this case, the unselected whole or partial portion may be regarded as a non-articulated structure by the system so that their movement may be prohibited. A closed shape drawn by the doctor through the input device that covers at least part of the controls of the model image may be obtained, and all parts contained (i.e., enclosed) in the shape are taken as the first portion. Such a design can improve the configuration efficiency of the first portion.

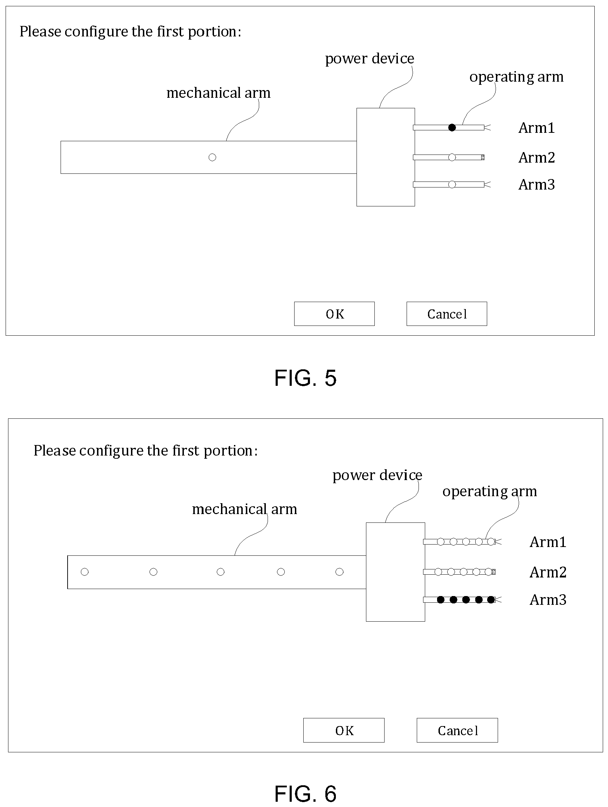

Referring to FIG. 5 and FIG. 6, FIG. 5 and FIG. 6 respectively illustrate the configuration interface for configuring the first portion of the surgical robot shown in FIG. 1 according to one embodiment. In FIG. 5 and FIG. 6, for example, a part that can be configured as at least part of the first portion can be represented by an icon control "O", a part that has been configured as at least part of the first portion can be represented by an icon control "●". Referring to FIG. 5, the model image basically illustrates the basic structure of the single-port surgical robot shown in FIG. 1, in which, the mechanism arm and the operating arms Arm1 to Arm3 respectively correspondingly include a control which is available for selection, and whether to select the control correspondingly determines whether to take the arm corresponding to the control as the first portion. For example, as shown in FIG. 5, only the whole operating arm Arm1 is selected to be configured as the first portion, and the end effector at the distal end of the operating arm Arm1 is configured as a controlled end effector. As shown in FIG. 6, the mechanism arm and the operating arms Arm1 to Arm3 in the model image respectively correspondingly include a plurality of controls which are available for selection, and the numbers of the controls in the mechanism arm and the operating arms Arm1 to Arm3 are basically the same as the numbers of the joint assemblies contained therein. Each control may, for example represent a corresponding joint. For example, as shown in FIG. 5, since all of the controls of the operating arm Arm3 are selected, so that it is equivalent to configuring the whole operating arm Arm3 as the first portion.

When applied to subsequent embodiments, the first portion configured by the doctor through the configuration interface may be obtained in advance, and then the purpose of the present disclosure may be achieved by using the first portion. Such a design can make it easier for the doctor to flexibly configure the expected first portion for the different scenarios.

Depending on the configuration, at least one end effector may be configured as the controlled end effector to be controlled by the motion input device.

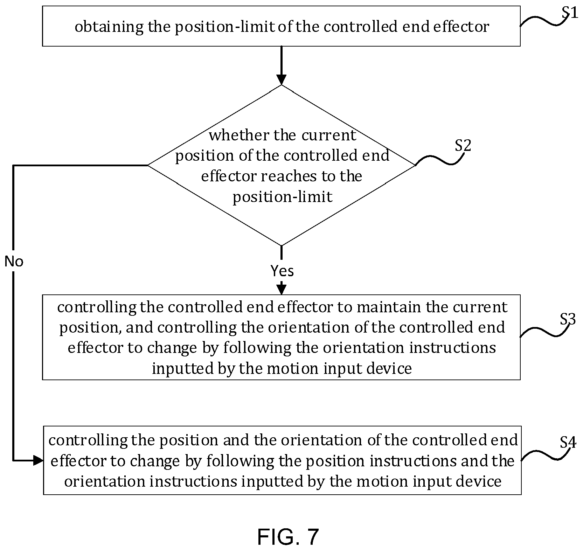

In one embodiment, a control method of the surgical robot is provided, and the control method may be executed by a controller. Referring to FIG. 7, the control method includes the following steps:
Step S1, obtaining the position-limit of the controlled end effector.
Step S2, determining whether the current position of the controlled end effector reaches the position-limit.

The position-limit is generally a range of values (also called a set of values), and "reaching" the position-limit generally means exceeding the range of values.

In step S2, specifically, when the current position of the controlled end effector reaches the position-limit, proceed to step S3; otherwise, proceed to step S4.

Step S3, controlling the controlled end effector to maintain the current position, and controlling the orientation of the controlled end effector to change by following the orientation instructions inputted by the motion input device.

In step S3, the orientation of the controlled end effector changes consistently with the orientation of the motion input device when the DoF(s) inputted by the motion input device corresponds with the DoF(s) of the movement of the controlled end effector, and the orientation of the controlled end effector changes inconsistently with the orientation of the motion input device when the DoF(s) inputted by the motion input device can not correspond with the DoF(s) of the movement of the controlled end effector. No matter what, the orientation of the controlled end effector substantively changes by following the orientation instructions inputted by the motion input device.

Step S4, controlling the position and the orientation of the controlled end effector to change by following the position instructions and the orientation instructions inputted by the motion input device.

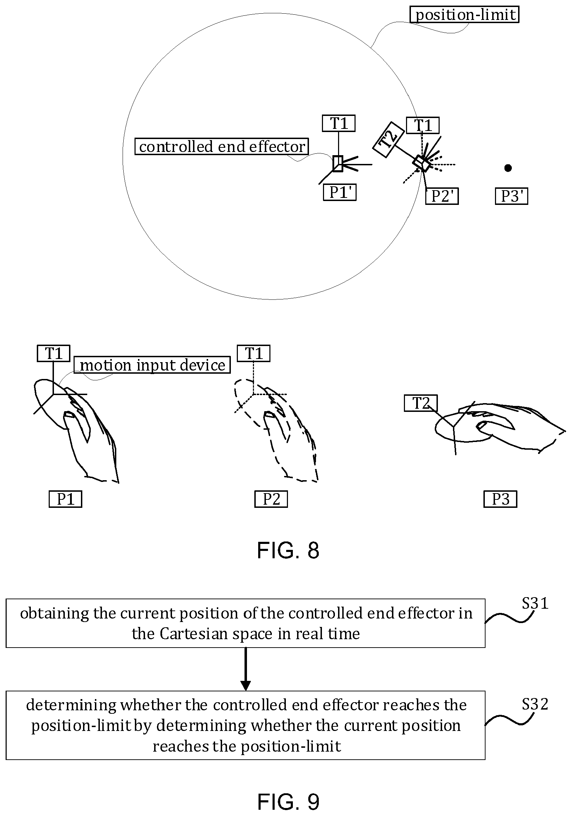

Referring to FIG. 8, for the purpose of concise, the first portion only illustrates the schematic structure of the controlled end effector, and the motion input device is also illustrated concisely. For example, the doctor holds the motion input device, which reaches the position P3 from the position P1 through the position P2. Besides, the orientation of the motion input device does not change during the process from the position P1 to the position P2, and the orientation is T1. And the orientation of the motion input device changes during the process from the position P2 to the position P3, and the orientation changes from T1 to T2.

Assuming that the limitations of the above steps S1 to S4 are not considered, the position of the controlled end effector may reach the position P3' from the position P1' through the position P2' corresponding to the motion input device moving to the position P3 from the position P1 through the position P2. Besides, the orientation of the controlled end effector with respect to the motion input device remains T1 when the controlled end effector moves from the position P1' to the position P2', and the orientation of the controlled end effector with respect to the motion input device changes from T1 to T2 when the controlled end effector moves from the position P2' to the position P3'.In this case, the moved distance of the motion input device and the moved distance of the controlled end effector usually keep a certain proportional relationship, and the moved distances of the two may be the same or different. In this case, the orientation of the motion input device is consistent with the orientation of the controlled end effector.

However, in the scenario where the limitations of the above steps S1 to S4 need to be considered, the movement process, more particularly, the positional change process of the controlled end effector needs to be monitored in real time. For example, the controlled end effector does not reach the position-limit when the controlled end effector moves from the position P1' to the position P2', so that the controlled end effector usually does not need to be restricted, that is, the controlled end effector moves freely by following the motion input device, both following in the position and following in the orientation. Since the position P2' and also the position P3' reach the position-limit when the controlled end effector moves from the position P2' to the position P3', so that the controlled end effector needs to be restricted, that is, the controlled end effector only follows the motion input device in the orientation and not in the position. In other words, the position of the controlled end effector is maintained and only the orientation of the controlled end effector is changed.

According to the above steps S1 to S4, by allowing the controlled end effector to move within the position-limit when the controlled end effector does not reach the position-limit, and by controlling the controlled end effector to maintain the position when the controlled end effector reaches the position-limit, the safety and reliability of the surgical operation can be ensured. And by controlling the orientation of the controlled end effector to change by following the orientation instructions inputted by the motion input device, since the motion input device always maintains the same orientation with the controlled end effector, so that it is not necessary to re-align the two in orientation, and the continuity in the surgical operation can be ensured. This is especially suitable for non-mechanical motion input devices, such as a magnetic navigation-type motion input device, that do not have the function of automatic aligning the orientations of the motion input device and the controlled end effector

In one embodiment, the position-limit of the controlled end effector may be the position-limit of a task space. The task space may be such as the Cartesian space. And then, referring to FIG. 9, the step S3, i.e., the step of determining whether the current position of the controlled end effector reaches the position-limit, includes:

Step S31, obtaining the current position of the controlled end effector in the task space in real time.

In Step S31, for example, the joint variables of each joint assembly in the first portion can be sensed by the sensors and the like, and then the current position of the controlled end effector in the reference frame of the Cartesian space may be calculated by combining the joint variables and the kinematic model corresponding to the first portion. These joint variables refer to the values of joint assemblies of the rotary joint assemblies and/or the offset values of the joint assemblies of the prismatic joint assemblies in the joint assemblies.

In this case, the kinematic model corresponding to the first portion of the actuating arm can be constructed by the controller in real time, for example, by obtaining all of the joint assemblies and the link parameters of the first portion, and then constructing the kinematic model corresponding to the first portion based on the link parameters. In one embodiment, the relevant information such as the link parameters for constructing the kinematic model of all the joint assemblies in the actuating arm may be stored in a storage unit of the operating arm. In another embodiment, the relevant information such as the link parameters for constructing the kinematic model of all the joint assemblies in the actuating arm except the joint assemblies of the operating arm may be stored in another storage unit which is different from the storage unit of the operating arm and coupled to the controller. Such another storage may be integrated in the controller or independent from the controller.

Step S32, determining whether the controlled end effector reaches the position-limit by determining whether the current position reaches the position-limit.

In step S32, the current position and the position-limit generally need to be compared in a same reference frame as the reference, which include situations that both positions are directly located in the reference frame, both are converted to be located in the reference frame, and one of them is located in the reference frame and the other one of them is converted to be located in the reference frame.

The reference frame may be set arbitrarily, for example, the reference frame may be defined as the base coordinate frame of the actuating arm. For another example, the reference frame can be defined as the tool coordinate frame of the actuating arm. In addition, it is feasible even to define a certain frame outside of the surgical robot as the reference frame.

In step S32, it can be determined that the controlled end effector has reached the position-limit when it is determined that the current position reaches the position-limit. Otherwise, it can be determined that the controlled end effector has not reached the position-limit.

In one embodiment, the above-mentioned position-limit may be a maximum range of motion in Cartesian space that can be obtained by the controlled end effector based on the coordinative movement of each joint assembly of the first portion. Furthermore, the above position-limit may also be a range of motion within the maximum range of motion which is determined based on the maximum range of motion. For example, it may be configured as a safe range of motion within the maximum range of motion.

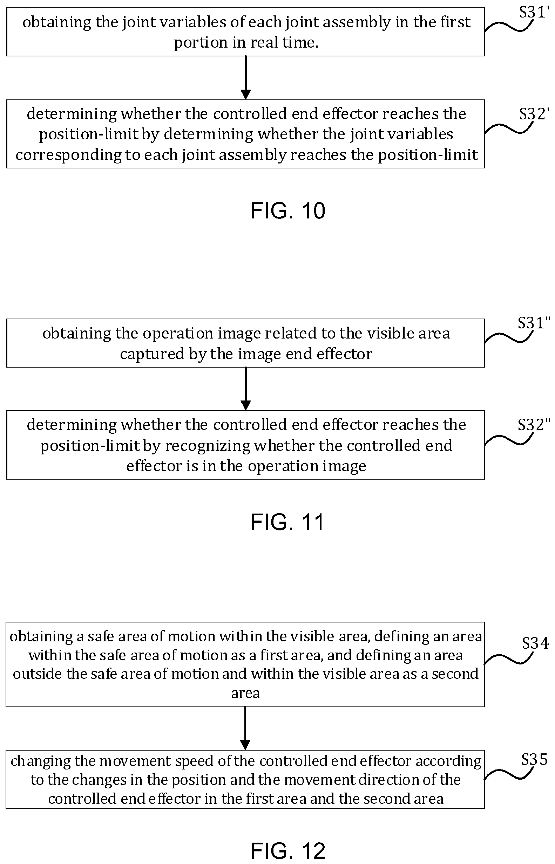

In another embodiment, since the changes in the pose of the controlled end effector are determined based on the movement of each joint assembly in the first portion, so that the above position-limit may also be a position-limit in joint space of each joint assembly in the first portion. Referring to FIG. 10, the above step S3, i.e., the step of determining whether the current position of the controlled end effector reaches the position-limit, includes:
Step S31', obtaining the joint variables of each joint assembly in the first portion in real time.
   The step S31' may obtain the required joint variables in various ways. For example, it may directly obtain the joint variables of the joint assemblies by the sensors arranged at each joint assembly. For another example, it may also obtain the current position of the controlled end effector in the reference frame, and then obtains the joint variables of each joint assembly in the first portion by resolving the current position by using the inverse kinematics. The current position may be obtained by measuring through the external sensor(s), and may also be obtained by resolving the target potion at the current time inputted by the motion input device.
Step S32', determining whether the controlled end effector reaches the position-limit by determining whether the joint variables corresponding to each joint assembly reaches the position-limit.

In step S32', it may be determined that the controlled end effector reaches the position-limit when it is determined that the joint variables of any one or more joint assemblies have reached the position-limit of the joint assemblies. Otherwise, it may be determined the controlled end effector does not reach the position-limit when it is determined that the joint variables of all joint assemblies do not reach the position-limit of the joint assemblies.

In this case, each joint assembly has a physical position-limit corresponding to itself, and such position-limit may be taken as the position-limit of the corresponding joint assembly.

For example, the position-limit in this embodiment may be the position-limit of each joint assembly in the first portion that affects the positional changes of the controlled end effector. In the above step S31', only the joint variables of the joint assemblies affecting the positional changes of the controlled end effector need to be determined, and then the joint variables may be compared with the corresponding position-limit in the above step S32'. In this case, the joint assemblies which affects the position of the controlled end effector can be accurately determined based on the configuration of the first portion, as this has generally been determined at the beginning of the design.

In one embodiment, the end effector may include at least an image end effector, and for the purpose of surgical operation, the end effector may also include an operation end effector. The controlled end effector may be configured as the image end effector. The controlled end effector may also be configured as the operation end effector.

In some embodiments, in case where the controlled end effector is configured to be the operation end effector, the position-limit may be determined based on the visible area of the image end effector in the reference frame. For example, the position-limit may be configured as the visible area of the image end effector in the reference frame. For another example, the position-limit may also be configured as an area within the visible area.

The camera parameters may generally include the field angle and the depth of field. The field angle is related to the focal length, and the depth of field is related to the aperture. In this case, the smaller the focal length, the larger the field angle, and the closer the visible distance; the larger the focal length, the smaller the field angle, and the farther the visible distance. In one embodiment, obtaining the visible area according to the camera parameters may be specifically obtaining the visible area based on the field angle and the depth of field. For example, the visible area can be calculated by using the trigonometric formula combined with the field angle and the depth of field. The visible area can be obtained by real-time calculation, or can be obtained directly from a preset database such as a comparison table according to the field angle and the depth of field.

In fact, according to the obtained visible area, a three-dimensional space or a plane area of the three-dimensional space may be obtained. For example, in case where the three-dimensional space is represented by f(x, y, z),the plane area corresponding to the depth of field z of the three-dimensional space may be represented by f(x, y). In this case, the f(x, y, z) can be converted to f'(x, y, z) in the reference frame by frame conversion,, and the f(x, y) can also be converted to f'(x, y) in the reference frame, so that the position range of the visible area in the reference frame can be obtained.

In case where the position-limit is the visible area of the image end effector in the reference frame, the accidental injury to the patient caused by the unexpected movement of the end effector outside of the visible area can be prevented effectively. Referring to FIG. 11, the step S3, i.e., the step of determining whether the current position of the controlled end effector reaches the position-limit, may also include:
Step S31", obtaining the operation image related to the visible area captured by the image end effector.
Step S32", determining whether the controlled end effector reaches the position-limit by recognizing whether the controlled end effector is in the operation image.

In order to facilitate image recognition in step S32", easily recognizable features can be set for the corresponding parts of at least the first portion to improve the recognition speed and accuracy. The step S32" may perform the image recognition by combining with a neural network such as a convolutional neural network. In step S32", whether a part is in the operation image can be recognized according to a preset strategy. For example, it is possible to determine whether the part is in the operation image by recognizing whether a certain specific point of the part locates in the operation image. For example, it is also possible to determine whether the part is in the operation image by recognizing whether a certain specific area of the part locates in the operation image. For example, it is also possible to determine whether the part is in the operation image by recognizing whether an overall outline of the part locates in the operation image. Such preset strategies can be set in advance, or can be selected according to the operation instructions inputted by the doctor during use.

In step S32", it can be determined that the controlled end effector does not reach the position-limit when it is recognized that the controlled end effector is located in the operation image. Otherwise, it can be determined that the controlled end effector reaches the position-limit.

In one embodiment, referring to FIG. 12, the step S3, specifically, the step of controlling the orientation of the controlled end effector to change by following the orientation instructions inputted by the motion input device, includes:

Step S34, obtaining a safe area of motion within the visible area, defining an area within the safe area of motion as a first area, and defining an area outside the safe area of motion and within the visible area as a second area.

Step S35, changing the movement speed of the controlled end effector according to the changes in the position and the movement direction of the controlled end effector in the first area and the second area.

In this case, the movement direction of the controlled end effector may generally be determined as follows: obtaining the current position of the controlled end effector, and obtaining the target position of the controlled end effector at the next moment, and then determining the movement direction of the controlled end effector according to the target position and the current position.

Further, the target position of the controlled end effector at the next moment may be obtained, for example, by the following steps: obtaining the target pose information inputted by the motion input device, calculating the joint variables of each joint assembly of the surgical arm according to the target pose information, obtaining the kinematic model of the surgical arm, and determining the target position of the target part at the next moment by combining the kinematic model and each of the joint variables.

The step S35, i.e., the step of changing the movement speed of the controlled end effector according to the changes in the position and the movement direction of the controlled end effector in the first area and the second area, may be realized as follows:

For example, decreasing the movement speed of the controlled end effector in the corresponding direction when the controlled end effector moves from the boundary of the first area to the outer boundary of the second area, and increasing the movement speed of the controlled end effector in the corresponding direction when the controlled end effector moves from the outer boundary of the second area to the boundary of the first area. In this case, the second area may include an inner boundary and the outer boundary, the inner boundary of the second area and the boundary of the first area may be the same, both refer to a boundary of the safe area of motion, and the outer boundary of the second area refers to a boundary of the visible area.

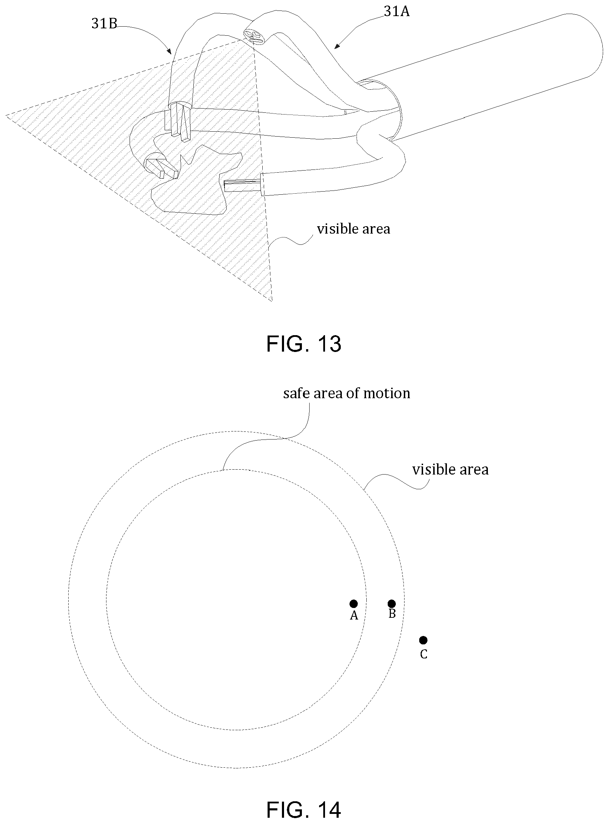

Referring to FIG. 13 and FIG. 14, a point A is located in the first area, a point B is located in the second area, and a point C is located outside the second area. The whole movement process of the controlled end effector such as the end effector moving from the point A to the point C through the point B, is divided into three stages, which include a first stage from the point A to the boundary of the first area, a second stage from the boundary of the first area to the outer boundary of the second area, and a third stage from the outer boundary of the second area to the point C. In this case, the movement speed in the first stage may be v1, the movement speed in the second stage may be v2, and the movement speed in the third stage may be v3, and v1 >v2>v3, where v3=0. That is, the whole movement process actually includes only the first stage and the second stage. Continuing to refer to FIG. 19, an whole movement process from the point C to the point A through the point B actually includes two stages, which are a first stage from the outer boundary of the second area to the boundary of the first area, and a second stage form the boundary of the first area to the point A. The movement speed in the first stage may be v1, and the movement speed in the second stage may be v2, at this time, v1 <v2.

In one embodiment, the movement speed of the controlled end effector in the corresponding direction is in positive correlation with the distance between the controlled end effector and the outer boundary of the second area. That is, the smaller the distance between the controlled end effector and the outer boundary of the second area, the smaller the movement speed; and the larger the distance between the controlled end effector and the outer boundary of the second area, the larger the movement speed. Generally, the movement speed is substantially equal to 0 when the controlled end effector reaches the boundary of the visible area and the movement direction is towards outside of the visible area. And the movement speed of the controlled end effector may substantially return to normal value when the controlled end effector reaches the boundary of the safe area of motion and the movement direction is away from the visible area.

Preferably, the movement speed of the controlled end effector in the corresponding direction is in a linear positive correlation with the distance between the controlled end effector and the outer boundary of the second area. Preferably, the movement speed of the controlled end effector in the corresponding direction is in an exponential positive correlation with the distance between the controlled end effector and the outer boundary of the second area. Such designs may enable the doctor to clearly feel that the controlled end effector is moving from the inner boundary of the second area to the boundary of the outer boundary.

In other embodiments, the controlled end effector may move at a first constant speed in the first area and move at a second constant speed in the second area. Generally, the first constant speed is greater than the second constant speed.

In some embodiments, the changes of the movement speed of the controlled end effector in the different areas and/or in the different movement directions may generally be the changes resulted from the overall changes of the movement speed of the first portion. For example, the movement speed of the controlled end effector may be changed by changing the ratio of the movement speed of the first portion. The ratio may be related to the area where the controlled end effector is located and the movement direction of the controlled end effector.

In some embodiments, the changes of the movement speed of the controlled end effector in the different areas and/or in the different movement directions may not be the changes resulted from the overall changes of the movement speed of the first portion. For example, the different movement speeds of the controlled end effector in different areas and/or in different movement directions can be obtained by solving in case where the DoFs of the first portion have sufficient redundancy compared to the DoFs required for realizing the expected task.

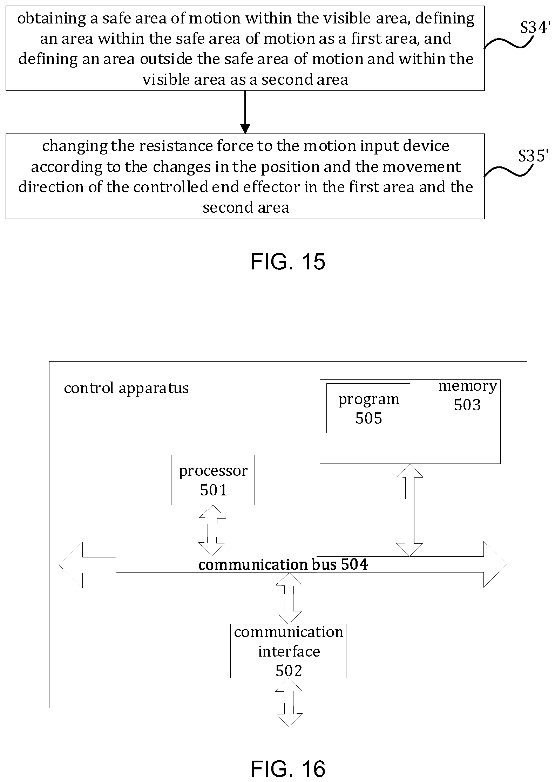

In one embodiment, the motion input device may be a mechanical motion input device, which includes a plurality of joint assemblies, sensors and drive motors, where the sensors are coupled to the controller and configured for sensing the states of each joint assembly, and the drive motors are coupled to the controller and configured for actuating the movement of each joint assembly. On the basis of the structure, referring to FIG. 15, the step S3, i.e., the step of restricting the controlled end effector to move within the visible area based on the visible area, may also include:
Step S34', obtaining the configured safe area of motion located in the visible area.
   For the convenience of description, in step S34', the visible area and the safe area of motion may also be divided into a first area and a second area as described above.
Step S35', changing the resistance force to the motion input device according to the changes in the position and the movement direction of the controlled end effector in the first area and the second area.

In this case, the step S35' mainly causes a drive motor to generate a reverse torque in the association direction based on the resistance force. The step S35', i.e., the step of changing the resistance force to the motion input device according to the changes in the position and the movement direction of the controlled end effector in the first area and the second area, may specifically be realized as follows:

For example, increasing the resistance force to the controlled end effector in the corresponding direction when the controlled end effector moves from the boundary of the first area to the outer boundary of the second area; and decreasing the resistance force to the controlled end effector in the corresponding direction when the controlled end effector moves from the outer boundary of the second area to the boundary of the first area.

In one embodiment, the resistance force to the motion input device moving in the corresponding direction is in negative correlation with the distance between the controlled end effector and the outer boundary of the second area. Generally, the movement speed is substantially equal to 0 when the controlled end effector reaches the boundary of the visible area and the movement direction is towards outside of the visible area. At this time, the resistance force is extremely large against the doctor to operate the motion input device, and the motion input device can hardly be moved by the doctor due to the relatively large resistance force, which can make the movement speed of the controlled end effector approach to 0. The movement speed of the controlled end effector may substantially return to normal when the controlled end effector reaches the boundary of the safe area of motion and the movement direction is away from the visible area.

Preferably, the resistance force to the motion input device moving in the corresponding direction is in a linear negative correlation with the distance between the controlled end effector and the outer boundary of the second area. Preferably, the resistance force to the motion input device moving in the corresponding direction is in an exponential negative correlation with the distance between the controlled end effector and the outer boundary of the second area. Such designs may enable the doctor to clearly feel that the controlled end effector is moving from the inner boundary of the second area to the boundary of the outer boundary and good force feedback can be achieved.

In other embodiments, the motion input device may move under a first constant resistance force when the controlled end effector moves in the first area. And the motion input device may move under a second constant resistance force when the controlled end effector moves in the second area. Generally, the second constant resistance force is greater than the first constant resistance force.

In one embodiment, a computer-readable storage medium can be provided, the computer-readable storage medium stores a computer program, and the computer program is configured to be loaded and executed by a processor to implement the following steps: obtaining position-limit of the controlled end effector; determining whether the controlled end effector reaches the position-limit; in case where the controlled end effector reaches the position-limit, controlling the controlled end effector to maintain the current position and controlling the orientation of the controlled end effector to change by following the orientation instructions inputted by the motion input device;, otherwise, controlling the position and the orientation of the controlled end effector to change by following the position instructions and the orientation instructions inputted by the motion input device.

In some embodiments, a control apparatus of the surgical robot is provided. As shown in FIG. 16, the control apparatus may include: a processor 501, a communication interface 502, a memory 503, and a communication bus 504.

The processor 501, the communication interface 502, and the memory 503 can communicate with each other through the communication bus 504.

The communication interface 502 is configured to communicate with network elements of other devices such as various sensors or motors or solenoid valves or other clients or servers, etc.

The processor 501 is configured to execute the program 505, and may specifically execute the corresponding steps in the embodiments of the method mentioned above.

Specifically, the program 505 may include program codes that include computer operation instructions.

The processor 505 may be a central processing unit (CPU), or an ASIC (Application Specific Integrated Circuit), or one or more integrated circuits configured to implement the embodiments of the present disclosure, or a GPU (Graphics Processing Unit). The one or more processors of the control apparatus may be the same type of processors, such as one or more CPU, or one or more GPU. The one or more processors of the control apparatus also may be different types of processors, such as one or more CPU, and one or more GPU.

The memory 503 is configured to store the program 505. The memory 503 may include a high-speed RAM memory, or may further include a non-volatile memory, such as at least one magnetic disk memory.

The program 505 may be specifically configured to make the processor 501 to execute the following operations: obtaining the position-limit of the controlled end effector; determining whether the controlled end effector reaches the position-limit; in case where the controlled end effector reaches the position-limit, controlling the controlled end effector to maintain the current position, and controlling the orientation of the controlled end effector to change by following the orientation instructions inputted by the motion input device; otherwise, controlling the position and the orientation of the controlled end effector to change by following the position instructions and the orientation instructions inputted by the motion input device.

The various technical features of the above-described embodiments may be combined in any combination. To make the description concise, not all possible combinations of the various technical features are described in the above-described embodiments. However, as long as the combination of these technical features does not conflict, it should be considered as falling into the scope of the present specification.

The above-described embodiments have only expressed several embodiments of the present application, which are described in detail and specifically, but are not therefore to be construed as limiting the scope of the present application. It should be noted that variations and modifications may be made to one of ordinary skill in the art without departing from the spirit of the present application, all of which fall within the scope of the present application. Therefore, the scope of the application should be subject to the accompanying claims.

## Claims

1. A control method of a surgical robot, the surgical robot comprising an actuating arm and a motion input device for manipulating changes in pose of a distal end of a first portion of the actuating arm, the distal end of the first portion comprising an end effector, the end effector comprising a controlled end effector currently configured to be manipulated by the motion input device, **characterized in that** the control method comprises steps of:
obtaining a position-limit of the controlled end effector;
determining whether a current position of the controlled end effector reaches the position-limit; and
controlling the controlled end effector to maintain the current position, and controlling an orientation of the controlled end effector to change by following an orientation instruction inputted by the motion input device, in case where the current position of the controlled end effector reaches the position-limit; otherwise, controlling the position and the orientation of the controlled end effector to change by following an position instruction and an orientation instruction inputted by the motion input device.

2. The control method according to claim 1, wherein, the position-limit is a position-limit in a task space, the step of determining whether the current position of the controlled end effector reaches the position-limit, comprises:
obtaining the current position of the controlled end effector in the task space in real time; and
determining whether the controlled end effector reaches the position-limit by determining whether the current position reaches the position-limit.

3. The control method according to claim 2, wherein, the position-limit is a maximum range of motion of the controlled end effector allowed by coordinative movements of respective joint assemblies in the first portion.

4. The control method according to claim 3, wherein, the position-limit is a range of motion within the maximum range of motion that is determined based on the maximum range of motion.

5. The control method according to claim 1, wherein, the changes in pose of the controlled end effector are determined according to movements of respective joint assemblies in the first portion, the position-limit is a position-limit of each assembly in the first portion in a joint space, the step of determining whether the current position of the controlled end effector reaches the position-limit comprises:
obtaining joint variables of each joint assembly in the first portion in real time; and
determining whether the controlled end effector reaches the position-limit by determining whether the joint variables corresponding to each joint assembly reach the position-limit.

6. The control method according to claim 5, wherein, the position-limit is a position limit of each joint assembly in the first portion that affects the change in position of the controlled end effector.

7. The control method according to claim 1, wherein, the controlled end effector is an image end effector.

8. The control method according to claim 1, wherein, the controlled end effector is an operation end effector.

9. The control method according to claim 8, wherein, the position-limit is determined based on a visible area of the image end effector in a reference frame.

10. The control method according to claim 9, wherein, the position-limit is the visible area of the image end effector in the reference frame.

11. The control method according to claim 9, wherein, the position-limit is an area within the visible area.

12. The control method according to claim 9, wherein, the visible area is determined based on camera parameters of the image end effector, and the camera parameters comprise a field angle and a depth of field.

13. The control method according to claim 12, wherein, the step of determining whether the controlled end effector reaches the position-limit comprises:
obtaining an operation image of the visible area captured by the image end effector; and
determining whether the controlled end effector reaches the position-limit by recognizing whether the controlled end effector is in the operation image.

14. The control method according to claim 12, wherein, the step of controlling the position and the orientation of the controlled end effector to change by following position instructions and orientation instructions inputted by the motion input device comprises:
obtaining a safe area of motion within the visible area, defining an area within the safe area of motion as a first area, and defining an area outside of the safe area of motion and within the visible area as a second area; and
changing movement speed of the controlled end effector according to the changes in position and in movement direction of the controlled end effector in the first area and the second area.

15. The control method according to claim 14, wherein, the step of changing the movement speed of the controlled end effector according to the changes in position and in movement direction of the controlled end effector in the first area and the second area comprises:
decreasing the movement speed of the controlled end effector in a corresponding direction in case where the controlled end effector moves from a boundary of the first area to an outer boundary of the second area; and increasing the movement speed of the controlled end effector in a corresponding direction in case where the controlled end effector moves from the outer boundary of the second area to the boundary of the first area.

16. The control method according to claim 15, wherein, the movement speed of the controlled end effector in the corresponding direction is in positive correlation with a distance between the controlled end effector and the outer boundary of the second area.

17. The control method according to claim 12, wherein, the motion input device is a mechanical motion input device, and the step of controlling the position and the orientation of the controlled end effector to change by following position instructions and orientation instructions inputted by the motion input device comprises:
obtaining a safe range of motion within the visible area, defining an area within the safe range of motion as a first area, and defining an area outside of the safe range of motion and within the visible area as a second area; and
increasing a resistance force against the controlled end effector moving in a corresponding direction in case where the controlled end effector moves from a boundary of the first area to an outer boundary of the second area; and decreasing the resistance force to the controlled end effector moving in the corresponding direction in case where the controlled end effector moves from the outer boundary of the second area to the boundary of the first area.

18. The control method according to claim 17, wherein, the resistance force against the motion input device moving in the corresponding direction is in negative correlation with the distance between the controlled end effector and the outer boundary of the second area.

19. The control method according to claim 1, wherein, the control method comprises:
obtaining description information of configuration of the actuating arm; and
generating a configuration interface for configuring the first portion based on the description information, the configuration interface comprising a control which is associated with structure of each part of the actuating arm.

20. The control method according to claim 19, wherein, the configuration interface comprises a model image generated based on the description information and associated with the actuating arm, and the model image comprises a control corresponding to each part of the actuating arm or a control corresponding to each joint assembly of each part of the actuating arm.

21. A computer-readable storage medium, **characterized in that,** the computer-readable storage medium stores a computer program, which is configured for being loaded and executed by a processor to implement the steps of the control method according to any one of claims 1 to 20.

22. A control apparatus of surgical robot, **characterized in that,** comprises:
a memory, configured for storing a computer program; and
a processer, configured for loading and executing the computer program;
wherein the computer program is configured for being loaded and executed by the processor to implement the steps of the control method according to any one of claims 1 to 20.

23. A surgical robot, **characterized in that,** the surgical robot comprises:
an actuating arm;
a motion input device for manipulating changes in pose of a distal end of a first portion of the actuating arm; and
a controller coupled to the actuating arm and the motion input device, and configured for implementing the steps in the control method according to any one of claims 1 to 20.

24. The surgical robot according to claim 23, wherein, the actuating arm comprises a mechanical arm and an operating arm, the operating arm is mounted to a distal end of the mechanical arm, the end effector is mounted to a distal end of the operating arm; and the first portion is the operating arm, or, the first portion is the mechanical arm and the operating arm.

25. The surgical robot according to claim 23, wherein, the actuating arm comprises a mechanical arm, an adjusting arm, a manipulator and an operating arm, a proximal end of the adjusting arm is mounted to a distal end of the mechanical arm, a proximal end of the manipulator is mounted to a distal end of the adjusting arm, a proximal end of the operating arm is mounted to a distal end of the manipulator, and the end effector is mounted to a distal end of the operating arm; the first portion is the operating arm, or, the first portion is the manipulator and the operating arm, or, the first portion is the mechanical arm, the adjusting arm, the manipulator and the operating arm.
